# EUROPÄISCHE PATENTSCHRIFT

(11) **EP 1 508 591 B1**
(45) Veröffentlichungstag und Bekanntmachung des Hinweises auf die Patenterteilung: **10.05.2006**
(21) Anmeldenummer: 03019044.1
(22) Anmeldetag: 22.08.2003
(51) Int. Cl.: C08L 3/02, A61K 9/48

(54) **Hüllmaterial für nahtlose Kapseln**
Wrapping material for seamless capsules
Matériau d'enrobage pour gélules sans soudure

(43) Veröffentlichungstag der Anmeldung: 23.02.2005
(73) Patentinhaber: Symrise GmbH & Co. KG, 37603 Holzminden (DE)
(72) Erfinder: Voigt, Ines, 22087 Hamburg (DE); Schleifenbaum, Birgit, 1299 Crans pres Celigny (CH); Aickele, Frank, 37603 Holzminden (DE)
(74) Vertreter: Eisenführ, Günther

(56) Entgegenhaltungen:
- EP-A- 0 389 700
- US-B1- 6 214 376
- US-B1- 6 375 981
- PATENT ABSTRACTS OF JAPAN vol. 1995, no. 11, 26. Dezember 1995 (1995-12-26) & JP 07 196478 A (HAJIME SUGII), 1. August 1995 (1995-08-01)

## Beschreibung

Die vorliegende Erfindung betrifft Zusammensetzungen, die als Hüllmaterial 1 für die Herstellung einer nahtlosen Kapsel mit einem flüssigen Kern in einer diesen Kern umgebenden Hülle eingesetzt werden können. Die Erfindung betrifft des Weiteren (a) entsprechende nahtlose Kapseln, (b) die Verwendung einer erfindungsgemäßen Zusammensetzung als Hüllmaterial in einem Mehrstoffdüsen-Verfahren mit eingetauchter Düse sowie (c) Verfahren zur Herstellung einer nahtlosen Kapsel, in denen die erfindungsgemäße Zusammensetzung eingesetzt wird.

Die erfindungsgemäßen Zusammensetzungen und die Hüllen der erfindungsgemäßen Kapseln zeichnen sich vorzugsweise dadurch aus, dass sie frei von Gelatine sind.

Gelatinehaltige Kapseln, wie sie aus dem Stand der Technik in großer Zahl bekannt sind, weisen eine Reihe von Nachteilen auf. Von Konsumenten werden sie häufig aus religiösen Gründen oder - z.B. von Vegetariern - auch aus ernährungsbedingten Gründen abgelehnt. Zudem stehen sie als tierische Produkte im Zusammenhang mit der Diskussion, die BSE (Bovine Spongiforme Enzephalopathie) betrifft, und sie werden dadurch zumindest potentiell von den Konsumenten weniger akzeptiert. Aus technologischer Sicht ist zudem zu bernerken, dass Gelatinekapseln zu einer unerwünschten statischen Aufladung neigen. Zudem führen bereits übliche Schwankungen von Luftfeuchte und Temperatur häufig zum unerwünschten Verkleben von Gelatinekapseln.

Es besteht deshalb ein genereller Bedarf an gelatinefreien, nahtlosen Kapseln, welche die folgenden Kriterien erfüllen sollten: Die Kapseln sollten (a) aus verzehrfähigen Zutaten bestehen, (b) sich im Mund lösen, (c) getrocknet werden können und (d) sich mittels eines Mehrstoffdüsen-Verfahrens herstellen lassen.

Nahtlose Kapseln mit einem flüssigen Kern und einer diesen Kern umgebenden Hülle sind bereits bekannt. Sie lassen sich insbesondere über ein Tropfverfahren mit Mehrstoffdüse herstellen, welches im Rahmen des vorliegenden Textes auch als Mehrstoffdüsen-Verfahren bezeichnet wird (vgl. Bauer, Frömmig, Führer; Pharmazeutische Technologie; Wissenschaftliche Verlagsgesellschaft mbH, Stuttgart, 6. Auflage, 1999, S. 347). Bezugnahmen auf das Mehrstoffdüsen-Verfahren sind dabei im Rahmen dieses Textes auch als Bezugnahme auf eine Vielzahl verwandter Verfahren zur Herstellung nahtloser Kapseln zu verstehen, soweit sich aus dem Textzusammenhang nicht im Einzelfall etwas anderes ergibt.

Im Mehrstoffdüsen-Verfahren werden Kapseln mit einer nahtlosen Hülle nach Art eines Tropfverfahrens hergestellt. Üblicherweise werden ein lipophiles Kernmaterial und eine warme hydrophile Hülllösung gleichzeitig durch eine konzentrische Mehrstoffdüse gepumpt, so dass sie in eine kalte lipophile Kühlflüssigkeit, beispielsweise ein Pflanzenöl, eintropfen. Die Düse kann dabei direkt in die Kühlflüssigkeit eintauchen (Submerged Nozzle-Verfahren).

Beim Eintropfen nehmen die Kapseln aufgrund der Grenzflächenspannungen eine Kugelform (sphärische Form) an. Aufgrund der Temperaturabsenkung bei Kontakt mit der Kühlflüssigkeit erstarrt die nahtlose Kapselhülle. Für weitere Ausführungen zur Durchführung des Mehrstoffdüsen-Verfahrens sei auf die umfangreiche Literatur verwiesen.

In den Patentschriften US 4,481,157 und US 4,251,195 werden Verfahren und Apparate zur kontinuierlichen Herstellung von nahtlosen Kapseln nach dem Mehrstoffdüsen-Verfahren beschrieben, bei denen die Düse in die Kühlflüssigkeit eintaucht. Diese Verfahren sind auch im Rahmen der vorliegenden Erfindung besonders bevorzugt.

EP 0 389 700 A1 beschreibt gelatinefreie Kapseln, deren Hülle Agar-Agar, Glycerin und Wasser enthält. Nachteilig an der offenbarten Hüllmaterial-Zusammensetzung ist, dass ihre Trockenmasse (bei alleiniger Verwendung von Agar-Agar und Glycerin neben Wasser) nicht auf einen Wert von etwa 15 Gew.-% oder mehr erhöht werden kann, wenn die Zusammensetzung im Mehrstoffdüsen-Verfahren verarbeitet werden soll. Betrüge der Anteil an Agar-Agar in der Zusammensetzung beispielsweise 10 Gew.-% oder mehr, würde die Viskosität der Gesamtzusammensetzung bereits derart hoch sein, dass ein Mehrstoffdüsen-Verfahren (Tropfprozess) nicht mehr möglich wäre. Die geringen im Rahmen der EP 0 389 700 A1 realisierbaren Trockenmassen (für die Verwendung in einem Mehrstoffdüsen-Verfahren) bedingen eine hohe Trocknungszeit der fertigen Kapsel.

JP 7 196 478 offenbart nahtlose gelatinefreie Kapseln, deren Hüllmaterial-Zusammensetzung die folgenden drei Komponenten umfasst: (1) ein essbares, wasserlösliches Polymer (z.B. Alginsäure, ein Alginat, Carrageen oder, Dextrin.), wobei die Viskosität einer 1%-igen Lösung des Polymers bei 70 °C 1000 cps oder weniger beträgt; (2) ein gelierender, filmbildender Agar auf natürlicher Basis und/oder zwei oder weniger niedermolekulare. Agare mit einer Gelstärke < 300 g/cm²; (3) einen Weichmacher wie Glycerin oder Sorbitol. Die Herstellung der Kapsel erfolgt im Tropfverfahren. Die in der JP 7 196 478 offenbarten Hüllmaterial-Zusammensetzungen besitzen jedoch eine nur geringe Trockenmasse von unter 10 Gew.-%, was wiederum hohe Trocknungszeiten bedingt. Es wird in der JP 7 196 478 nicht offenbart, wie die Trockenmasse der Hüllmaterial-Zusammensetzung auf einen Wert von beispielsweise 15 Gew.-% erhöht werden kann, ohne dabei die Fähigkeit der Zusammensetzung zur Verwendung im Mehrstoffdüsen-Verfahren zu beeinträchtigen. Es werden ferner keine Kapseln offenbart, deren Kapselhülle ohne das Risiko einer Beeinträchtigung der Stabilität getrocknet werden kann.

US 6,214,376 offenbart gelatinefreie Kapseln, zu deren Herstellung Hüllmaterial-Zusammensetzungen eingesetzt werden, die (1) 8 bis 50 Gew.-% Weichmacher, (2) 0,5 bis 12 Gew.-% kappa-Carrageenan und (3) 1 bis 95 Gew.-% Wasser enthalten. Hydrolysierte Stärken wie z.B. Maltodextrin können zur Erhöhung der Trockenmasse eingesetzt werden. Carrageen ist gemäß der technischen Lehre der US 6,214,376 B1 der hauptsächliche oder alleinige Gelbildner in den offenbarten Zusammensetzungen. Mit Formulierungen, die Carrageen als (alleinigen oder hauptsächlichen) Gelbildner enthalten, ist jedoch der Nachteil verbunden, dass die Gele ihre maximale Festigkeit beim Eintreten in ein Fällbad nur langsam erreichen, wenn dieses Fällbad keine Kationen- umfasst. Für die weiteren Verarbeitungsschritte (Zentrifugieren, Trocknen) wäre eine Kapselhülle, die Carrageen als hauptsächlichen oder alleinigen Gelbildner umfasst, daher ohne Härtung in Anwesenheit von Ionen nicht ausreichend stabil. In der US 6,214,376 wird konsequenter Weise das Mehrstoffdüsen-Verfahren nicht als Möglichkeit angegeben, die offenbarten Kapseln herzustellen. Verwiesen wird lediglich auf das "Rotary Die"-Verfahren.

US 6,375,981 offenbart ebenfalls gelatinefreie Kapseln. Die Kapselmaterial-Zusammensetzung umfasst (1) ein Stärkeprodukt mit einem Dextrose-äquivalent kleiner als 1, (2) ein Pflanzengummi (vorzugsweise kappa-Carrageenan) und (3) einen Weichmacher. Die offenbarten Zusammensetzungen besitzen jedoch hohe Viskositäten und sind deshalb nicht zur Verwendung in einem Mehrstoffdüsen-Verfahren zur Herstellung nahtloser Kapseln geeignet. Diese hohen Viskositäten werden dabei durch die eingesetzten Stärkeprodukte verursacht, die bereits in geringen Konzentrationen (in einer wässrigen Hüllmaterial-Zusammensetzung) die Viskosität in der beschriebenen Weise beeinflussen.

Es war deshalb die Aufgabe der vorliegenden Erfindung, alternative gelatinefreie Zusammensetzungen anzugeben, die als Hüllmaterial für die Herstellung einer nahtlosen Kapsel mit einem flüssigen Kern und einer diesen Kern umgebenden Hülle eingesetzt werden können. Vorzugweise sollte die Viskosität der Zusammensetzung so gewählt sein, dass die Herstellung der Kapsel in einem Mehrstoffdüsen-Verfahren möglich ist; vorteilhafterweise sollten dabei die Düsen einer einzusetzenden Mehrstoffdüsen-Anordnung in das zugehörige Härtungsbad eintauchen können, ohne dass dies die Qualität der herzustellenden Kapseln beeinträchtigt. Die Trockenmasse der anzugebenden Hüllmaterial-Zusammensetzung sollte vorzugsweise mindestens 15 Gew.-% betragen, damit nach Herstellung der Kapsel unter Verwendung der anzugebenden Hüllmaterial-Zusammensetzungen die Kapselhülle getrocknet werden kann, ohne dass es hierbei zu einer Beeinträchtigung der Kapselstabilität kommt. Die anzugebende Hüllmaterial-Zusammensetzung sollte zudem vorteilhafterweise nur geringe Anteile an potentiell kariogenen Substanzen wie Mono- und Disacchariden umfassen. Besonders vorteilhafterweise sollte die Viskosität der anzugebenden Hüllmaterial-Zusammensetzung bei 80 °C zwischen 60 und 150 mPas liegen. Bei Temperaturen zwischen 25 °C und 50 °C sollte sich aus der Hüllmaterial-Zusammensetzung ein festes Gel bilden lassen. Es versteht sich, dass vorteilhafterweise mehrere der vorgenannten Eigenschaften durch die anzugebende Hüllmaterial-Zusammensetzung erreicht werden sollten.

Überraschenderweise haben eigene Untersuchungen nun gezeigt, dass eine Zusammensetzung, die
- 1,5 - 4 Gew.-% Agar
- 10 - 22 Gew.-% einer hydrolysierten Stärke mit einer Viskosität < 50 mPas, gemessen bei 80 °C in wässriger Lösung mit einem Anteil von 15 Gew.-% an der hydrolysierten Stärke, bezogen auf die Gesamtmasse der wässrigen Lösung,
- 70 - 85 Gew.-% Wasser sowie
- gegebenenfalls einen oder mehrere übliche Zusatzstoffe,
umfasst, wobei die Gewichtsprozentangaben, soweit nicht anders angegeben, auf die Gesamtmasse der Zusammensetzung bezogen sind (dies gilt für den gesamten vorliegenden Text), die gestellte(n) Aufgabe(n) löst.

Das Agar übernimmt in der erfindungsgemäßen Zusammensetzung die Aufgabe des (primären) Gelbildners; wie weiter unten noch im Detail ausgeführt werden wird, ist die Anwesenheit weiterer Gelbildner jedoch möglich und manchmal vorteilhaft.

Die hydrolysierte Stärke mit einer Viskosität < 50 mPas wird als Füllstoff eingesetzt, um die Trockenmasse der Zusammensetzung auf einen hohen Wert (> 15 Gew.-%) einzustellen, ohne dabei die Gesamt-Viskosität der Zusammensetzung in nachteiliger Weise zu erhöhen. Die hydrolysierte Stärke besitzt vorzugsweise keine nennenswerten hygroskopischen Eigenschaften, ist geschmacksneutral, besitzt ein vernachlässigbares kariogenes Potential und ist wasserlöslich.

Weitere Aspekte der vorliegenden Erfindung betreffen (a) nahtlose Kapseln, welche eine erfindungsgemäße Hüllmaterial-Zusammensetzung umfassen, (b) die Verwendung einer erfindungsgemäßen Zusammensetzung als Hüllmaterial in einem Mehrstoffdüsen-Verfahren mit eingetauchter Düse sowie (c) Verfahren zur Herstellung einer nahtlosen Kapsel unter Verwendung einer Mehrstoffdüsen-Anordnung.

Bevorzugte Ausgestaltungen der Erfindung ergeben sich aus der nachfolgenden Beschreibung sowie den beigefügten Patentansprüchen.

Vorteilhafterweise ist das in der erfindungsgemäßen Zusammensetzung eingesetzte Agar so beschaffen, dass es eine Viskosität im Bereich zwischen 0,5 und 5 mPas, vorzugsweise im Bereich zwischen 1 und 4 mPas besitzt, wobei die Viskosität bei 80 °C in wässriger Lösung mit einem Anteil von 1 Gew.-% des Agar, bezogen auf die Gesamtmasse der wässrigen Lösung, gemessen wird.

Vorteilhafterweise besitzt das Agar dabei eine Gelstärke im Bereich von 600 bis 900 g/cm², wobei eine Gelstärke im Bereich von 650 bis 750 g/cm² besonders favorisiert wird, da bei höheren Gelstärken die Viskosität in wässriger Lösung bei gleicher Agar-Konzentration etwas höher ist, was zu einer nachteiligerweise etwas früheren Gelierung führt. Eine Agar-Qualität, welche die bevorzugten Eigenschaften erfüllt ist "Quick Soluble Agar N700" von C. E. Roeper.

Bevorzugt ist überdies eine Konzentration des Agar in einer erfindungsgemäßen Zusammensetzung, die im Bereich von 3 bis 4 Gew.-% liegt.

Es sei darauf hingewiesen, dass Agar ein Polysaccharid ist, das aus Rotalgen gewonnen wird. Eine Agar-Lösung geht erst bei etwa 60 °C vom Gel- in den Sol-Zustand über, der Übergang vom Sol- in den Gel-Zustand (Gelierpunkt) erfolgt jedoch erst bei etwa 30 °C. Diese große Differenz zwischen Gelier- und Schmelztemperatur ist eine besondere Eigenschaft des Agar im Vergleich zu den meisten anderen Polysaccharid-Gelen; sie ist auch dafür verantwortlich, dass Agar im Rahmen der vorliegenden Erfindung als (primärer) Gelbildner eingesetzt wird. Das Gelierverhalten von Agar ist besonders vorteilhaft. Im Gegensatz z.B. zu der relativ langsamen Gelbildung des Carrageenans härtet ein Agargel bei Geliertemperatur innerhalb weniger Sekunden durch und bietet dadurch der gebildeten Kapsel eine hohe Stabilität für die Weiterverarbeitung.

Die erfindungsgemäße Zusammensetzung kann gegebenenfalls einen oder mehrere übliche Zusatzstoffe enthalten.

Insbesondere ist es in manchen Fällen vorteilhaft, neben Agar (welches wie erwähnt als primärer Gelbildner fungiert) einen weiteren Gelbildner einzusetzen. Vorteilhafterweise handelt es sich hierbei um kappa-Carrageenan, ein Polysaccharid, das aus Rotalgen extrahiert wird. Zur Bildung eines Gels auf Basis von kappa-Carrageenan ist die Anwesenheit bestimmter Kationen, beispielsweise Kalium-Ionen, erforderlich; Gele auf Basis von kappa-Carrageenan sind thermoreversibel. Die Viskosität einer Carrageenan-Lösung steigt exponentiell mit der Konzentration an Carrageen an; sie ist außerdem abhängig vom Carrageen-Typ.

Im Rahmen der vorliegenden Erfindung hat sich der Einsatz einer kappa-Carrageenan-Qualität mit einer Viskosität im Bereich von 3 mPas bis 10 mPas und vorzugsweise einer Gelstärke von 700 bis 1000 g/cm² als bevorzugt herausgestellt; die Viskosität wurde dabei bei 80 °C in wässriger Lösung mit einem Anteil von 1 Gew.-% an kappa-Carrageenan bestimmt. Durch den zusätzlichen Einsatz von kappa-Carrageenan wird ein besonders festes Gel gebildet, wodurch die Hüllstabilität vor allem im noch nicht getrockneten Zustand wesentlich erhöht wird.

Besonders bevorzugt als Co-Gelbildner in einer erfindungsgemäßen Zusammensetzung ist eine kappa-Carrageenan-Qualität mit einer Viskosität im Bereich von 5 mPas bis 8 mPas (Messmethode wie zuvor) und einer Gelstärke von 800 bis 900 g/cm². Eine Qualität, welche diese Eigenschaften erfüllt, ist "Gelcarin® 812" von FMC Biopolymer.

Die vorstehenden Angaben zur Gelstärke von Agar und kappa-Carrageenan beziehen sich hierbei auf die "Nikkansui Measuring Method". Bei dieser Bestimmungsmethode wird eine 1,5 gew.-%ige Lösung der zu untersuchenden Substanz in Wasser hergestellt. Diese Lösung wird 15 Std. bei 20 °C gelagert. Anschließend wird das erhaltene Gel mit Gewichten belastet. Die Gelstärke in g/cm² ist die Maximalmasse, bei der das belastende Gewicht während eines Zeitraumes von 20 sek. nicht in das Gel eindringt.

Kappa-Carrageenan wird in den erfindungsgemäßen Zusammensetzungen vorteilhaft in Konzentrationen im Bereich zwischen 0,01 und 1 Gew.%, bevorzugt in Mengen im Bereich von 0,1 bis 0,9 Gew.-%, weiter bevorzugt in Mengen im Bereich von 0,5 bis 0,9 Gew.-% eingesetzt.

Das Massenverhältnis Agar : sonstige Getbildrier sollte für erfindungsgemäße Zusammensetzungen im Bereich von 1,5 : 1 bis 400 : 1 liegen, insbesondere wenn kappa-Carrageenan als Co-Gelbildner eingesetzt wird. In diesem Falle liegt vorteilhafterweise jedoch das Massenverhältnis Agar : kappa-Carrageenan im Bereich von 3 :1 bis 25 : 1.

Hydrolysierte Stärken wie die in der erfindungsgemäßen Zusammensetzung eingesetzte Stärke werden aus nativer Stärke durch partielle enzymatische oder Säure-Hydrolyse hergestellt. Dabei werden die Stärke-Polymerketteri teilweise abgebaut. Hydrolysierte Stärkeprodukte lassen sich durch ihren DE-Wert beschreiben. Dieser DE-Wert (DE = Dextrose-Equivalent) ist dimensionslos und entspricht dem Massenprozentanteil an reduzierendem Zucker, berechnet als Glucose. Je höher der DE-Wert einer hydroysierten Stärke, desto geringer ist im Allgemeinen die Länge der darin vorhandenen Polymerketten- und desto geringer ist auch die Viskosität. Hydrolysierte Stärken mit einem DE-Wert im Bereich von 5 bis 20 werden auch als Maltodextrine bezeichnet; zu den hydrolysierten Stärken zählen überdies auch die Dextrine. Die Stärken, aus denen hydrolysierte Stärken hergestellt werden, können aus unterschiedlichen pflanzlichen Rohstoffquellen stammen; insbesondere kommen als Rohstoffquelle Mais, Weizen, Tapioka und Kartoffel in Betracht.

Es wurde bereits erwähnt, dass hydrolysierte Stärken, die in einer erfindungsgemäßen Zusammensetzung eingesetzt werden, eine Viskosität < 50 mPas besitzen; bevorzugt sind sie jedoch hydrolysierte Stärken mit einer Viskosität von unter 10 mPas (wobei die Viskosität wie bereits angegeben bestimmt wird).

In einer erfindungsgemäßen Zusammensetzung werden die hydrolysierten Stärken in einem Anteil von 10 bis 22 Gew.-% eingesetzt; bevorzugt sind jedoch Konzentrationen im Bereich von 15 bis 20 Gew.-%.

Gemäß einer besonders bevorzugten Ausgestaltung der vorliegenden Erfindung umfasst die erfindungsgemäße Zusammensetzung 0,02 bis 2 Gew.-% Gummi Arabicum. Gummi Arabicum ist ein Gemisch von Polysacchariden, das aus Acacia-Arten gewonnen wird; wässrige Lösungen bilden keine Gele. Im Rahmen der Arbeiten zur vorliegenden Erfindung hat sich überraschenderweise gezeigt, dass die Zugabe von Gummi Arabicum zu der erfindungsgemäßen Hüllmaterial-Zusammensetzung einer unerwünschten Satellitenbildung bei der Kapselausformung im Mehrstoffdüsen-Verfahren entgegenwirkt. Wenngleich die Gründe hierfür noch nicht vollständig bekannt sind, lässt sich doch vermuten, dass der Anteil an Gummi Arabicum die viskoelastischen Eigenschaften der erfindungsgemäßen Hüllmaterial-Zusammensetzung zur Anwendung im Mehrstoffdüsen-Verfahren optimiert. Offenbar führen geringe Anteile an Gummi Arabicum (Anteile im angegebenen Bereich) zu einer Absenkung der Viskosität. Höhere Mengen an Gummi Arabicum in einer erfindungsgemäßen Zusammensetzung führen allerdings zu einer unerwünschten Herabsetzung der Gelstärke der erstarrten Hüllmaterial-Zusammensetzung. Gummi-Arabicum-Konzentrationen im Bereich von 0,02 bis 2 Gew.-% sind innerhalb der erfindungsgemäßen Zusammensetzung bevorzugt, wobei der Bereich zwischen 0,2 und 0,8 Gew.-% als besonders bevorzugt bezeichnet werden kann.

Die erfindungsgemäße Zusammensetzung umfasst vorteilhafterweise einen Weichmacher, der der Hüllmaterial-Zusammensetzung zugegeben wird, um die Sprödigkeit der getrockneten Kapselhülle (nach Herstellung der Kapsel und Trocknung) herabzusetzen. Vorteilhafterweise sind dabei Konzentrationen im Bereich von 0,5 bis 10 Gew.-%, vorzugweise 1,5 bis 5 Gew.-%, wiederum bezogen auf die Gesamtmasse der erfindungsgemäßen Zusammensetzung. Bezogen auf die Trockenmasse der Zusammensetzung liegen bevorzugte Weichmacheranteile im Bereich von 2 bis 40 Gew.-%, wobei Anteile zwischen 2 und 25 Gew.-% bevorzugt und Anteile im Bereich von 5 bis 15 Gew.-% besonders bevorzugt sind. Als Weichmacher werden vorzugsweise Substanzen eingesetzt, die aus der Gruppe ausgewählt sind, bestehend aus: Glycerin, Propylenglykol, Sorbitol, Mannitol und deren Mischungen. Andere mehrwertige Alkohole sowie andere Substanzen, welche in der beschriebenen Weise die Sprödigkeit der getrockneten Kapselhülle- herabsetzen, können aber natürlich ebenfalls als Weichmacher eingesetzt werden.

Als Mittel zur Erhöhung der Geliertemperatur und somit auch als Mittel zur Unterstützung der Gelbildung in der Hüllmaterial-Zusammensetzung können insbesondere chemisch modifizierte Stärken eingesetzt werden, beispielsweise Stärke-Ester. Geeignete chemisch modifizierte (z.B. veresterte) Stärken besitzen eine Viskosität im Bereich von 0,5 bis 1,5 mPas, vorzugsweise im Bereich von 0,8 bis 1,2 mPas, gemessen bei 80 °C in wässriger Lösung mit einem Anteil von 1 Gew.-% chemisch modifizierter Stärke. Vorzugsweise liegt der Anteil der besagten modifizierten Stärken in einer erfindungsgemäßen Zusammensetzung im Bereich von 0,5 bis 2 Gew.-%, bevorzugt im Bereich von 0,8 bis 1,2 Gew-%, wiederum bezogen auf die Gesamtmasse der erfindungsgemäßen Zusammensetzung.

Die Viskosität der erfindungsgemäßen Zusammensetzung wird vorzugsweise über die Auswahl der Bestandteile und ihrer Konzentrationen auf einen Wert im Bereich zwischen 20 und 150 mPas eingestellt, gemessen bei 80 °C; eine Viskosität in diesem Bereich ermöglicht eine problemfreie Anwendung der üblichen Mehrstoffdüsen-Verfahren. Bevorzugt sind Viskositäten im Bereich von 60 mPas bis 100 mPas.

Die erfindungsgemäßen Zusammensetzungen besitzen vorteilhafterweise einen Gelierpunkt im Bereich zwischen 25 und 50 °C, vorzugsweise zwischen 30 und 40 °C.

Es sei darauf hingewiesen, dass insbesondere bei der Kapselausformung über den Submerged Nozzle Prozess die Viskosität und das Gelierverhalten der erfindungsgemäßen Hüllmäterial-Zusammensetzung besonders beachtet werden müssen. Eine zu niedrige Viskosität oder ein zu niedriger Gelierpunkt verhindem eine ausreichend feste Kapselhülle im nassen Zustand. Die gebildeten Kapseln würden deshalb im Verlaufe weiterer Prozessschritte, wie z.B. beim Zentrifugieren, mit hoher Wahrscheinlichkeit mechanisch zerstört. Eine zu hohe Viskosität und/oder ein zu hoher Gelierpunkt verhindern andererseits eine korrekte Kapselausformung und verursachen zudem eine unerwünscht starke Satellitenbildung.

Die Viskosität der erfindungsgemäßen Hüllmaterial-Zusammensetzungen lässt sich mit einem Rheometer CVO 120 (Fa. Bohlin Instruments GmbH; Pforzheim) bestimmen. Als Messsystem dient hierbei ein Platte-Platte-System mit einem Plattendurchmesser von 50 mm. Es wird hierbei in Rotation gemessen; die Scherrate beträgt 1000 s⁻¹, der Spalt wird auf 500 µm eingestellt. Es wird isotherm gemessen, wobei die Temperatur bei 80 °C liegt.

Der Gelierpunkt der erfindungsgemäßen Hüllmaterial-Zusammensetzungen kann ebenfalls vorteilhaft mit einem Rheometer CVO 120 (Fa. Bohlin Instruments GmbH, Pforzheim) bestimmt werden. Als Messsystem dient wiederum ein Platte-Platte-System mit einem Plattendurchmesser von 50 mm. Es wird in Oszillation gemessen. Die Frequenz, beträgt konstant 1 Hz, der Spalt wurde auf 2500 µm eingestellt, die Temperatur wurde von 80 °C auf 10 °C mit einem Gradienten von 5 °C/min. abgesenkt. Als Gelierpunkt (Sol-Gel-Übergangspunkt), wurde die Temperatur abgelesen, bei der Viskositäts- oder Speichermodul G' gleich dem Elastizitäts- oder Verlustmodul G" ist. Vergleiche hierzu die Ausführungen in Thomas Mezger, Das Rheologie Handbuch, 2000.

Die erfindungsgemäße Hüllmaterial-Zusammensetzung kann neben den bereits angegebenen Bestandteilen auch einen oder mehrere Süßstoffe und/oder Farbstoffe umfassen.

Als Süßstoff verwendbar sind insbesondere Sucralose, Aspartam, Acesulfam, NA-Saccharin, Thaumatin und Neohesperidin. Als Farbstoffe können insbesondere wasserlösliche Lebensmittelfarbstoffe zugesetzt werden.

Es versteht sich, dass das in der erfindungsgemäßen Zusammensetzung vorhandene Wasser nach Herstellung einer Kapsel in einem Trocknungsschritt im Wesentlichen aus der Kapselhülle entfernt wird. Eine gewisse Restmenge an Wasser verbleibt jedoch regelmäßig gebunden im Hydrokolloid-Netzwerk der Kapselhülle.

Eine erfindungsgemäße nahtlose Kapsel umfasst-einen flüssigen Kern und eine feste Kapselhülle.

Eine erste erfindungsgemäße nahtlose Kapsel ist in einem Mehrstoffdüsen-Verfahren mit in ein Härtungsbad eingetauchter Düse herstellbar, wobei (a) eine flüssige Kernmischung und (b) als Hüllmaterial eine erfindungsgemäße flüssige Zusammensetzung eingesetzt werden.

Eine zweite erfindungsgemäße nahtlose Kapsel umfasst in der Kapselhülle
- 5 bis 26 Gew.-% Agar (vorzugsweise 10 bis 20 Gew.-%)
- 40 bis 75 Gew.% einer hydrolysierten Stärke mit einer Viskosität < 50 mPas, gemessen bei 80 °C in wässriger Lösung mit einem Anteil von 15 Gew.-% an der hydrolysierten Stärke, bezogen auf die Gesamtmasse der wässrigen Lösung,
- 0 bis 40 Gew.-% Weichmacher (vorzugsweise 2 bis 25 Gew.-%; besonders bevorzugt 5 bis 15 Gew.-%), sowie
- gegebenenfalls Restwasser (typischerweise 1 bis 5 Gew.-%, bezogen auf die Gesamtmasse der Kapselhülle) und einen oder mehrere übliche Zusatzstoffe,
wobei die Gewichtsprozentangaben, soweit nicht anders angegeben, auf die Trockenmasse der Hülle bezogen sind.

Der Anteil an kappa-Carrageenan in besonders bevorzugten erfindungsgemäßen Kapseln liegt im Bereich von 0,1 bis 4 Gew.-%. Der Anteil an Gummi Arabicum in besonders bevorzugten erfindungsgemäßen Kapseln liegt im Bereich von 0,08 bis 8 Gew.-%.

Hinsichtlich des flüssigen Kerns ist zu bemerken, dass dieser vorteilhafter Weise (a) eine sensorisch wirksame Menge an einem oder mehreren Aromastoffen und/oder (b) eine pharmazeutisch wirksame Menge an einem oder mehreren pharmazeutischen Wirkstoffen umfasst.

Der Anteil an Aromastoffen im flüssigen Kern liegt vorteilhafterweise zwischen 1 und 100 Gew.-%, bezogen auf die Gesamtmasse des flüssigen Kerns. Weitere vorteilhafte Ausgestaltungen des flüssigen Kerns und der Kernflüssigkeit, welche diesen bildet, ergeben sich aus den nachfolgenden Ausführungen:

Verfahrensbedingt muss die Kernflüssigkeit hydrophob sein. Beim Verzehr von großen Kapseln gelangt bei hohem Kemanteil eine relativ große Flüssigkeitsmenge direkt in den Mund. Dabei soll ein möglichst starker und unmittelbarer Aromaeindruck hervorgerufen werden.

Die Kernflüssigkeit kann eine Vielzahl unterschiedlicher Stoffe enthalten wie beispielsweise Konservierungsmittel, Antioxidantien, Verdünnungsmittel, Zucker, Zuckeraustauschstoffe, Zuckeralkohole, Süßstoffe, Genusssäuren, Farbstoffe, Färbemittel, Pigmente, geschmacksverstärkene Mittel, Geschmackstoffe, Aromen, etherische Öle, kühlende und/oder erfrischende Substanzen, Nutraceuticals, pharmazeutische Wirkstoffe, antimikrobielle Wirkstoffe, entzündungshemmende Substanzen, zahnpflegende Substanzen, Enzyme, pH-Regulatoren, Spurenelemente, Mineralien, Vitamine, fette Öle, Silikonöle, Fette, Verdünnungsmittel oder pflanzliche Extrakte.

Bevorzugt umfasst die Kernflüssigkeit mindestens ein fettes Öl und mindestens eine geschmackgebende Komponente, beispielsweise einen Geschmackstoff, ein Aroma oder ein etherisches ÖI.

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Pekannussöl, Pistazienkernöl, Rapsöl, Reiskeimöl, Sesamöl, Sojaöl, Sonnenblumenöl, Walnussöl oder Weizenkeimöl. Bevorzugt werden fraktionierte Kokosöle verwendet, die hauptsächlich Fettsäurereste C6-C8 aufweisen. Diese zeichnen sich durch ihre Geschmacksneutralität sowie durch ihre gute Oxidationsstabilität aus.

Der Aromaanteil richtet sich nach der Kapselgröße und gewünschter Geschmacksintensität und reicht üblicherweise von 1 bis 100 Gew.-%, bevorzugt 5 bis 90 Gew.-%, besonders bevorzugt 25 bis 75 Gew.-%, bezogen auf die gesamte Kemflüssigkeit. Im Sinne der vorliegenden Erfindung ist der Aromaanteil die Summe aller geschmacksgebenden Komponenten in der Kernflüssigkeit inklusive Süßstoffe und Stoffe, die den Trigeminus anregen (z.B. scharf schmeckende Stoffe oder solche mit kühlender oder wärmeerzeugenden Wirkung). Im wesentlichen geschmacksneutrale Stoffe, wie beispielsweise Pflanzenöle, werden demnach nicht zum Aromaanteil gezählt.

Beispiele für Geschmackstoffe oder Aromen, die Bestandteil des Kernmaterials sein können, finden sich z.B. in K. Bauer, D. Garbe, H. Surburg, Common Fragrance and Flavor Materials, 4^{th}. Ed., Wiley-VCH, Wemheim 2001 oder auch in S. Arctander, Perfume and Flavor Chemicals, Vol. I und 11, Montclair, N. J., 1969, Selbstverlag.

Als Beispiele für natürliche Aromen, die in der Kernflüssigkeit enthalten sein können, seien genannt: Pfefferminzöle, Krauseminzöle (Spearmintöle), Mentha-Arvensis-Öle, Anisöle, Nelkenöle, Citrusöle, Campheröle, Zimtöle, Zimtrindenöle, Wintergrünöle, Cassiaöle, Davanaöle, Fichtennadelöle, Eucalyptusöle, Eucalyptus-citriodora-Öle, Fenchelöle, Galbanumöle, Ingweröle, Kamillenöle, Kümmelöle, Citronellöle, Limetteöle, Orangenöle, Bergamottöle, Grapefruitöle, Mandarinenöl, Rosenöle, Geraniumöle, Salbeiöle, Scharfgarbenöle, Sternanisöle, Basilikumöl, Bittermandelöle, Thymianöle, Wacholderbeeröle, Rosmarinöle, Angelikawurzelöle, Vanilleextrakte, sowie Fraktionen davon bzw. daraus isolierte Inhaltsstoffe.

Als Beispiele für einheitliche Aromastoffe, die in der Kernflüssigkeit enthalten sein können, seien genannt: Anethol, Menthol, Menthon, Isomenthon, Menthylacetat, Menthofuran, Mintlacton, Eucalyptol (1,8-Cineol), Limonen, Eugenol, Thymol, Pinen, Sabinenhydrat, 3-Octanol, Carvon, gamma-Octalacton, gamma-Nonalacton, Germacren-D, Viridiflorol, 1,3E,5Z-Undecatrien, Isopulegol, Piperiton, 2-Butanon, Ethylformiat, 3-Octylacetat, Isoamylisovalerianat, Hexanol, Hexanal, cis-3-Hexenol, Linalool, alpha-Terpineol, cis- und trans- Carvylacetat, p-Cymol, Damascenon, Damascone, Rosenoxid, Fenchol, Acetaldehyddiethylacetal, 1-Ethoxyethylacetat, cis-4-Heptenal, Isobutyraldehyd, Isovaleraldehyd, cis-Jasmon, Methyldihydrojasmonat, Anisaldehyd, Methylsalicylat, 2'-Hydroxypropiophenon, Myrtenylacetat, 2-Phenylethylalkohol, 2-Phenylethylisobutyrat, 2-Phenylethylisovalerat, Zimtaldehyd, Geraniol, Nerol. Bei chiralen Verbindungen können die Aromastoffe als Racemat oder als einzelnes Enantiomer oder als enantiomerenangereichertes Gemisch vorliegen.

Verbindungen mit physiologischem Kühleffekt, die Bestandteil der Kernflüssigkeit sein können, sind beispielsweise 1-Menthol, Menthonglycerinacetal, Menthyllactat, substituierte Menthyl-3-carbonsäureamide (z.B. Menthyl-3-carbonsäure-N-ethylamid), 2-lsopropyl-N,2,3-trimethylbutanamid, substituierte Cyclohexancarbonsäureamide, 3-Menthoxypropan-1,2-diol, 2-Hydroxyethylmenthylcarbonat, 2-Hydroxypropylmenthylcarbonat, N-Acetylglycinmenthylester, Menthylhydroxycarbonsäureester (z.B. Menthyl-3-hydroxybutyrat), Monomenthylsuccinat 2-Mercaptocyclodecanon, Menthyl-2-pyrrolidin-5-oncarboxylat, bevorzugt sind 1- Menthol, Menthonglycerinacetal, Menthyllactat, Menthyl-3-carbonsäure-N-ethylamid, 3-Menthoxypropan-1,2-diol, 2-Hydroxyethylmenthylcarbonat, 2-Hydroxypropylmenthylcarbonat, Monomenthylsuccinat, Menthyl-2-pyrrolidin-5-oncarboxylat.

Vorteilhafte etherische Öle, die Bestandteil der Kernflüssigkeit sein können, sind beispielsweise Anisöl, Basilikumöl, Bittermandelöl, Campheröl, Cassiaöl, Citronellöl, Citrusöle, Eucalyptus-citriodora-Öl, Eucalyptusöl, Kamillenöl, Krauseminzöl (Spearmintöl), Limetteöl, Mandarinenöl, Nelkenöl, Orangenöl, Pfefferminzöl, Salbeiöl, Thymianöl, Wintergrünöle, Zimtöl, Zimtrindenöl.

Daneben sind besonders Kernflüssigkeiten geeignet mit kühlender und/oder erfrischender Wirkung im Mund-, Rachen- oder Nasenraum. Vorteilhafte Stoffe sind hier beispielsweise Menthol, Menthon, Carboxamide, Methonacetale, Mentholcarbonate, Mentholsuccinate, 1,8-Cineol (Eucalyptol), Carvon, alpha-Terpineol, Methylsalicylat, 2'-Hydroxypropiophenon, Menthylmethylether. Die optisch aktiven Verbindungen können in enantiomerenreiner Form eingesetzt werden oder als beliebige Gemische der Enantiomere.

Bevorzugt sind ebenfalls fruchtige Aromakompositionen der Geschmacksrichtungen Apfel, Birne, Pfirsich, Traube, Erdbeere, Himbeere, Kirsche, Ananas, Maracuja, Banane, Aprikose, Citrone, Orange, Grapefruit.

Die Kernflüssigkeit kann Verbindungen enthalten, die ein Wärme-, Schärfe-, Kribbel- oder Prickelgefühl auf der Haut oder auf den Schleimhäuten hervorrufen, insbesondere Aromastoffe mit einem wärmeerzeugenden Effekt und/oder scharf schmeckende Verbindungen (Scharfstoffe), wie beispielweise Capsaicin, Dihydrocapsaicin, Gingerol, Paradol, Shogaol, Piperin, Paprikapulver, Chili-Pfeffer-Pulver, Extrakte aus Paprika, Extrakte aus Pfeffer; Extrakte aus Chili-Pfeffer; Extrakte aus Ingwerwurzeln, Extrakte aus Aframomum melgueta, Extrakte aus Spilanthes-acmella, - Extrakte aus Kaempferia galanga, Extrakte aus Alpinia galanga, Carbonsäure-N-Vanillylamide, insbesondere Nonansäure-N-vanillylamid, 2-Nonensäureamide, insbesondere 2-Nonensäure-N-isobutylamid, 2-Nonensäure-N-4-hydroxy-3-methoxyphenylamid, Alkylether von 4-Hydroxy-3-methoxybenzylalkohol, insbesondere 4-Hydroxy-3-methoxybenzyl-n-butylether, Alkylether von 3-Hydroxy-4-methoxybenzylalkohol, Alkylether von 3,4-Dimethoxybenzylalkohol, Alkylether von 3-Ethoxy-4-hydroxybenzylalkohol, Alkylether von 3,4-Methylendioxybenzylalkohol, (4-Hydroxy-3-methoxyphenyl)essigsäureamide, insbesondere (4-Hydroxy-3-methoxyphenyl)essigsäure-N-n-octylamid, Nicotinaldehyd, Methylnicotinat, Propylnicotinat, 2-Butoxyethylnicotinat, Benzylnicotinat, 1-Acetoxychavicol.

Die Kernflüssigkeit kann vorteilhafterweise Stoffe zur Verbesserung der Mundhygiene enthalten, wie beispielsweise zahnpflegende und/oder erfrischende Stoffe. Zu den Stoffen zur Verbesserung der Mundhygiene zählen beispielsweise Stoffe zu Bekämpfung oder Verhinderung von Plaque, Zahnstein oder Karies sowie solche zur Bekämpfung oder Verhinderung von Mundgeruch. Es sei in diesem Zusammenhang auf die US 5,043,154 hingewiesen. Beispielhaft seien genannt Zn-Salze, wie Zn-citrat, Zn-fluorid, Sn-Salze, wie Sn-fluoride, Cu-Salze, Fluoride, z.B. Aminfluoride, Alkalifluoride wie Na-fluorid, Erdalkalifluoride, Ammoniumfluorid, Phosphate, Pyrophosphate, Fluorphosphate, wie Na-monofluorphosphat, Al-mono- und Al-difluorphosphat, alpha-lonone, Geraniol, Thymol, Eugenol, Isomenthylacetat, Panthenol (Provitamin B₅), Xylitol, Allantoin, Niacinamid (Vitamin B₃), Tocopherylacetat (Vitamin E - Actetat), Poloxamer.

Die Kernflüssigkeit kann, beispielsweise zur Verbesserung der Mundhygiene, antimikrobielle Wirkstoffe enthalten. Diese Wirkstoffe können hydrophiler, amphoterer oder hydrophober Natur sein. Als Beispiele seien genannt: Triclosan, Chlorhexidine, Peroxide, Phenole, Domiphenbromid (Phenododeciniumbromid), Bromchlorophen, Zn-Salze, Cu-Salze, quarternäre Monoammonium Salze wie Cocoalkylbenzyldimethylammoniumchlorid oder Cetylpyridiniumchlorid. Neben Einzelwirkstoffen können Mischungen von Wirkstoffen oder natürliche Extrakte bzw. Fraktionen hiervon enthaltend Wirkstoffe eingesetzt werden, wie z.B. solche erhältlich aus Neem, Berberitze, Fenchel, Grüntee, Ringelblume, Kamille, Rosmarin, Thymian, Propolis oder Gelbwurz.

Die Kernflüssigkeit kann pharmazeutische Wirkstoffe, wie beispielsweise Antibiotika, enthalten. Diese Wirkstoffe können hydrophiler, amphoterer oder hydrophober Natur sein. Als Beispiele seien genannt: β-Lactam-Wirkstoffe und deren Salze, Lactone, 2-Pyridone und 2-Pyrithone, Ciprofloxacin, Norfloxacin, Tetracycline, Erythromycin, Amikacin, Deoxycycline, Capreomycin, Chlortetracycline, Oxytetracycline, Clindamycin, Ethambutol, Metronidazol, Pentamidin, Gentamicin, Kanamycin, Lineomycin, Methacycline, Minocycline, Neomycin, Netilmicin, Paromomycin, Streptomycin, Tobramycin, Miconazol, Amanfadin.

Vorzugsweise werden die Antioxidantien gewählt aus der Gruppe bestehend aus Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-Carotin, Lycopin) und deren Derivate, Flavonoide, Quercetin, phenolische Benzylamine, Propylgallat, Octylgallat, Dodecylgallat, Liponsäure und deren Derivate (z.B. Dihydroliponsäure), Aurothiglucose, Propylthiouracil, Dilaurylthiodipropionat, Distearylthiodipropionat, (Metall)-Chelatoren (z.B. α-Hydroxyfettsäuren, Fettsäuren (Palminsäuren), Phytinsäure, Lactoferrin, EDTA, EGTA), α-Hydroxysäuren (z.B. Zitronensäure, Milchsäure, Apfelsäure), Bilirubin, ungesättigte Fettsäuren, und deren Derivate (z.B. Palmitoleinsäure, α-Linolensäure, γ-Linolensäure, Linolsäure, Ölsäure), Folsäure und deren Derivate, Ubichinon und Ubichinol und deren Derivate, Vitamin C und Derivate (z.B. Ascorbylpalmitat, Mg - Ascorbylphosphat, Ascorbylacetat), Tocopherole und Derivate (z.B. Vitamin E - acetat), Vitamin A und Derivate (Vitamin A - palmitat) Rutinsäure und deren Derivate, Ferulasäure und deren Derivate, Butylhydroxytoluol (BHT), Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z.B. ZnO, ZnSO₄), Selen und dessen Derivate (z.B. Selenmethionin), Stilbene und deren Derivate (z.B. Stilbenoxid, trans-Stilbenoxid) und geeignete Derivate (Salze, Ester, Ether, Zucker, Nukleotide, Nukleoside, Peptide, und Lipide) dieser genannten Wirkstoffe; aus Pflanzen isolierte Inhaltsstoffe, Extrakte bzw. Fraktionen davon z.B. aus Tee, Grüntee, Algen, Traubenkernen, Weizenkeimen, Kamille, Rosmarin, Oregano.

Als Konservierungsmittel können beispielsweise organische Säuren bzw. deren Salze eingesetzt werden, insbesondere Sorbinsäure, Benzoesäure, Ameisensäure und die Na-, K- und Ca-Salze dieser Säuren, sowie 4-Hydroxybenzoesäureester, Salicylsäure und Dehydracetsäure.

Als typische Zucker, die Bestandteil der Kernflüssigkeit sein können, seien Glucosesirupe, Glucose-Fructose Sirupe, Isomerose-Sirupe, Isoglucosesirupe, Invertzuckersirupe und kristalline Zucker wie Saccharose, Glucose, Lactose, hydrolisierte Lactose, Sorbose, Arabinose, Xylose, . Mannose, Maltose, Galactose, Maltotriose oder Fructose genannt.

Geeignete Zuckeraustauschstoffe sind Zuckeralkohole wie beispielsweise Mannit, Sorbit und Sorbitsirup, Isomalt (z.B. Palatinit® ), Maltit und Maltitsirup, Laktit, Xylit, Erythrit, Leucrose, Arabinol, Arabitol, Adonitol, Alditol, Ducitol, Iditol, Oligofructose oder Polydextrose.

Als typische Süßstoffe, die Bestandteil der Kernflüssigkeit sein können, seien Saccharin (gegebenenfalls als Na-, K- oder Ca-Salz), Aspartam (z.B: NutraSweet® ), Cyclamat (gegebenenfalls als Na- oder Ca-Salz), Acesulfam-K (z.B. Sunett® ), Thaumatin oder Neohesperidin-Dihydrochalkon genannt. Es können natürlich auch andere Süßstoffe wie Steviosid, Rebaudiosid A, Glycyrrhizin, Ultrasüß, Osladin, Brazzein, Miraculin, Pentadin, Phyllodulcin, Dihydrochalcone, Arylharnstoffe, trisubstituierte Guanidine, Glycyrrhizin, Superaspartam, Suosan, Sucralose (Trichlorgalactosaccarose, TGS), Alitam, Monellin oder Neotame® verwendet werden.

Die Kernflüssigkeit kann Genusssäuren enthalten wie beispielsweise Citronensäure, Adipinsäure, Äpfelsäure, Fumarsäure, Bernsteinsäure, Milchsäure, Essigsäure, Gluconsäure, Weinsäure. Die Säuren können zum Teil adstringierend und/oder mundwässernd bzw. speichelfördernd wirken.

Den Kernflüssigkeit kann beispielsweise folgende Farbstoffe, Färbemittel oder Pigmente enthalten: Lactoflavin (Riboflavin), beta-Carotin, Riboflavin-5'-phosphat, alpha-Carotin, gamma-Carotin, Zuckerkulör, Cantaxanthin, Erythrosin, Kurkumin, Chinolingelb, Gelborange S, Tartrazingelb, Bixin, Norbixin (Annatto, Orlean), Capsanthin, Capsorubin, Lycopin, beta-Apo-8'-Carotinal, beta-Apo-8'-Carotinsäureethylester, Xantophylle (Flavoxanthin, Lutein, Kryptoxanthin, Rubixanthin, Violaxanthin, Rodoxanthin), Echtes Karmin (Karminsäure, Cochenille), Azorubin, CocheniOerot A (Ponceau 4 R), Beetenrot, Betanin, Anthocyane, Amaranth, Patentblau V, Indigotin I (Indigo-Karmin), Chlorophylle, Kupferverbindungen der Chlorophylle, Brillantsäuregrün BS (Lisamingrün), Brillantschwarz BN, Carbo medicinalis vegetabilis, Titandioxid, Eisenoxide und -hydroxide, Calciumcarbonat, Aluminium, Silber, Gold, Rubinpigment BK (Litholrubin BK), Methylviolett B, Viktoriablau R, Viktoriablau B, Acilanbrillantblau FFR (Brillantwollblau FFR), Naphtolgrün B, Acilanechtgrün 10 G (Alkaliechtgrün 10 G), Ceresgelb GRN, Sudanblau II, Ultramarin, Phtalocyaninblau, Phtalocayaningrün, Echtsäureviolett R. Es können weitere, natürlich gewonnene Extrakte (z.B. Paprikaextrakt, Schwarzmöhrenextrakt, Rotkohlextrakt) zu Färbezwecken verwendet werden.

Als geschmacksverstärkende Mittel können in der Kernflüssigkeit Stoffe enthalten -sein wie Natrium- und Kaliumsalze (z.B. Natriumchlorid, Kaliumchlorid) und Geschmacksverstärker eingesetzt, wie z.B. Maltol, Furaneol zur Verbesserung des Süßgeschmackes, als auch Natrium-L-glutamat (MSG, Glutaminsäure), Inosin-5'-monophosphat (IMP), 5-Guanosinmonophosphat (GMP), hydrolisierte vegetabile Proteine (HVP), Hefeextrakte, Aminosäuren.

Die Kernflüssigkeit kann darüberhinaus gegebenenfalls ernährungsphysiologisch wirksame Stoffe oder Stoffgemische enthalten (Nutraceuticals). Es seien beispielsweise genannt Vitamin A und Derivate, Carotine, Vitamin C (Ascorbinsäure), Vitamin E (Tocopherol) und Derivate, Vitamine der B- und D-Reihe wie Vitamin B₆ (Nicotinamid), Vitamin B₁₂, Vitamin D₁, Vitamin D₃, Vitamin F, Folsäure, Vitamin K, Biotin, Aminosäuren, Verbindungen der Elemente Magnesium, Silicium, Phosphor, Calcium, Mangan, Eisen oder Kupfer, Enzyme wie z.B. Bromelain, ungesättigte Fettsäuren, ω-3-Fettsäuren, mehrfach ungesättigte Fettsäuren, γ-Linolensäure, Ölsäure, Eicosapentaensäure, Docosahexaensäure und deren Derivate, Bisabolol, Thymol, Extrakte oder andere Produkte pflanzlicher und tierischer Herkunft, z. B. Aprikosenöl, Avocadoöl, Babassuöl, Baumwollsamenöl, Aloe Vera Öl, gamma-Oryzanol, Hagebuttenkernöl, Hanföl, Johannisbeerkernöl, Jojobaöl, Kirschkernöl, Nachtkerzenöl, Nerzöl, Teebaumöl, Traubenkernöl, Fischöle, Lebertran, Ceramide und Ceramid-ähnliche Verbindungen, Pflanzenextrakte wie z. B. Arnika, Aloe, Bartflechte, Efeu, Brennessel, Ginseng, Henna, Kamille, Ringelblume, Rosmarin, Salbei, Schachtelhalm oder Thymian.

Die Kernflüssigkeit kann Verdünnungsmittel enthalten, wie beispielsweise Pflanzenöl-Triglyceride, 1,2-Propylenglykol, Benzylalkohol, Triacetin (Glycerintriacetat), Diazetin (Glycerindiacetat), Triethylcitrat, Glycerin oder Acetyl-triethylcitrat. Bevorzugte Verdünnungsmittel sind Pflanzenöl-Triglyceride und Triacetin oder Gemische enthaltend diese Verdünnungsmittel.

Ein weiterer Aspekt der Erfindung liegt in der Bereitstellung eines Verfahrens zur Herstellung einer erfindungsgemäßen nahtlosen Kapsel mit folgenden Schritten:
- Bereitstellen eines flüssigen Kernmaterials,
- Bereitstellen einer flüssigen erfindungsgemäßen Zusammensetzung,
- Simultanes Extrudieren des flüssigen Kernmaterials und der flüssigen Zusammensetzung durch die innere bzw. äußere Düse einer Mehrstoffdüsen-anordnung, so dass sich Tropfen bilden, die einen flüssigen Kern und eine flüssige Hülle besitzen,
- Härten der Tropfenhülle in einem Härtungsbad, so dass die Kapsel gebildet wird,
- gegebenenfalls Trocknen.

Vorzugsweise ist das erfindungsgemäße Herstellungsverfahren derart ausgestaltet, dass die Düse(n) der Mehrstoffdüsenanordnungen in das Härtungsbad eintauchen und dieses keine Kationen zur Aushärtung der flüssigen Zusammensetzung enthält.

Die Erfindung wird nachfolgend anhand von Ausführungsbeispielen näher erläutert.

### A. Herstellverfahren - allgemeine Angaben:

Die Bestandteile einer erfindungsgemäßen Hüllmaterial-Zusammensetzung werden zusammengegeben und im Wasserbad auf 90 °C erhitzt, bis eine klare, möglichst luftblasenfreie Lösung entstanden ist.

Die einzusetzende Kernflüssigkeit wird bei 20 °C bereitgestellt.

Hüllmaterial-Zusammensetzung und Kernflüssigkeit werden über ein Pumpensystem einer konzentrischen Zweistoffdüse zugeführt. Die. Leitung für die Hüllmaterial-Zusammensetzung wird dabei auf 60 bis 80 °C. gehalten. Die konzentrische Zweistoffdüse taucht in ein Bad aus flüssigem Pflanzenöl ein. Die Temperatur dieses Ölbades liegt unter 1.5 °C. Der aus der Zweistoffdüse in das Ölbad austretende Flüssigkeitsstrahl zerfällt, unterstützt durch zusätzliche Schwingungsanregung der Flüssigkeit, zu Einzeltropfen, die aus Kernflüssigkeit und flüssiger Hüllmaterial-Zusammensetzung gebildet sind (Kern und Hülle).

Nach Aushärtung werden die noch nassen Kapseln mittels Zentrifugation von anhaftendem Öl getrennt und anschließend unter ständiger Bewegung in einem trockenen Luftstrom getrocknet. Hierzu können übliche Wirbelbetttrockner oder Trommeltrockner verwendet werden; Voraussetzung ist hierbei jedoch, dass die Kapseln durch Rotation bzw. Luftverwirbelung in Bewegung gehalten werden können.

Alternativ können die Kapseln auch durch Zugabe von Kieselsäure in einem Mischprozess mit anschließender Siebung getrocknet werden.

### B. Beispiele für erfindungsgemäße Kapseln unter Angabe der Hüllmaterial-Zusammensetzung:

Es wurden insgesamt 11 verschiedene Kapseln hergestellt und in der nachfolgenden Tabelle die Parameter Kapseldurchmesser, Zusammensetzung Hüllmaterial, Viskosität Hüllmaterial-Zusammensetzung bei 80 °C, Gelierpunkt Hüllmaterial-Lösung, Trockensubstanz der Hüllmaterial-Zusammensetzung und Zusammensetzung der getrockneten Hülle (Trockensubstanz) eingetragen.

Alle in der Tabelle enthaltenen Prozentangaben sind Gewichtsprozente und beziehen sich auf die jeweilige Gesamtmasse der Hüllmaterial-Zusammensetzung bzw. der getrockneten Hülle. Die Restwassermenge in einer getrockneten Hülle kann je nach Trocknungsgrad und Umgebungsfeuchte zwischen 1 und 5 Gew.-% schwanken.

### Beispiele 1 - 11:

| Nr. | Kapsel durch-messer trocken [mm] | Zusammensetzung Hüllmaterial | | Viskosität Hüllmaterial-Zusammensetzung bei 80°C [mPas] | Gelierpunkt Hüllmateriallösung [°C] | Trockensubstanz der Hüllmaterialzusammensetzung [Gew.%] | Zusammensetzung der getrockneten Hülle (Trockensubstanz) |
|---|---|---|---|---|---|---|---|
| 1 | 2 | 3,5 | % Agar N700 | 56 | 34 | 23 | 15,5% |
| | | 16,5 | % Dextrin Crystal Tex 626 | | | | 70,7% |
| | | 2,5 | % Glycerin. | | | | 10,7% |
| | | 77,5 | % Wasser | | | | 3,1% |
| | | | | | | | |
| 2 | 2 | 3,5 | % Agar N700 | 30 | 34 | 23 | 15,5% |
| | | 16 | % Dextrin Crystal Tex 626 | | | | 69,0% |
| | | 2,5 | % Glycerin | | | | 10,7% |
| | | 0,5 | % Gummi Arabicum | | | | 1,9% |
| | | 77,5 | % Wasser | | | | 2,9% |
| | | | | | | | |
| 3 | 2 | 3,5 | % Agar N700 | 34 | 34 | 25 | 13,6% |
| | | 11 | % Dextrin Crystal Tex 626 | | | | 42,8% |
| | | 10 | % Glycerin | | | | 38,9% |
| | | 0,5 | % Gummi Arabicum | | | | 2,0% |
| | | 75 | % Wasser | | | | 2,7% |
| | | | | | | | |
| 4 | 2 | 3,5 | % Agar N700 | 26 | 36 | 25 | 13,6% |
| | | 11 | % Dextrin Crystal Tex 626 | | | | 42.7% |
| | | 10 | % Sorbitol | | | | 38,8% |
| | | 0,5 | % Gummi Arabicum | | | | 1,9% |
| | | 0,005 | % Acesulfam K | | | | 0,02% |
| | | 0,005 | % Aspartam | | | | 0,02% |
| | | 74,99 | % Wasser | | | | 2,96% |
| | | | | | | | |
| 5 | 2 | 3,5 | % Agar N700 | 100 | 39 | 23 | 15,6% |
| | | 14,75 | % Dextrin Crystal Tex 626 | | | | 62,8% |
| | | 0,5 | % Modifizierte Stärke Perfectagel MPT | | | | 2,0% |
| | | 0,75 | % Carrageen Gelcarin GP 812 | | | | 2,9% |
| | | 2,5 | % Glycerin | | | | 10,6% |
| | | 0,5 | % Gummi Arabicum | | | | 2.0% |
| | | 0,06 | % Kaliumchlorid | | | | 0,3% |
| | | 77,44 | % Deionisiertes Wasser | | | | 3,8% |
| | | | | | | | |
| 6 | 4 | 3,5 | % Agar N700 | 100 | 39 | 23 | 15,6% |
| | | 14,75 | % Dextrin Crystal Tex 626 | | | | 62,7% |
| | | 0,5 | % Modifizierte Stärke Perfectagel MPT | | | | 2,0% |
| | | 0,75 | % Carrageen Gelcarin GP 812 | | | | 2,9% |
| | | 2,5 | % Glycerin | | | | 10,6% |
| | | 0,5 | % Gummi Arabicum | | | | 2,0% |
| | | 0,06 | % Kaliumchlorid | | | | 0,3% |
| | | 77,44 | % Deionisiertes Wasser | | | | 3,9% |
| | | | | | | | |
| 7 | 2 | 3,5 | % Agar N700 | 102 | 44 | 23 | 15,6% |
| | | 14,25 | % Dextrin Crystal Tex 626 | | | | 61,0% |
| | | 1 | % Modifizierte Stärke Perfectagel MPT | | | | 3,9% |
| | | 0,75 | % Carrageen Gelcarin GP 812 | | | | 3,0% |
| | | 2,5 | % Glycerin | | | | 10,7% |
| | | 0,5 | % Gummi Arabicum | | | | 2,0% |
| | | 0,06 | % Kaliumchlorid | | | | 0,3% |
| | | 77,44 | % Deionisiertes Wasser | | | | 3,5% |
| | | | | | | | |
| 8 | 5 | 3,5 | % Agar N700 | 102 | 44 | 23 | 15,6% |
| | | 14,25 | % Dextrin Crystal Tex 626 | | | | 60,9% |
| | | 1 | % Modifizierte Stärke Perfectagel MPT | | | | 3,9% |
| | | 0,75 | % Carrageen Gelcarin GP 812 | | | | 3,0% |
| | | 2,5 | % Glycerin | | | | 10,7% |
| | | 0,5 | % Gummi Arabicum | | | | 2,0% |
| | | 0,06 | % Kaliumchlorid | | | | 0,3% |
| | | 77,44 | % Deionisiertes Wasser | | | | 3,6% |
| | | | | | | | |
| 9 | 1 | 3 | % Agar N700 | 60 | 33 | 23 | 12,7% |
| | | 16 | % Dextrin Crystal Tex 626 | | | | 69,6% |
| | | 0,5 | % Carrageen Gelcarin GP 812 | | | | 2,0% |
| | | 2,5 | % Glycerin | | | | 10,8% |
| | | 0,5 | % Gummi Arabicum | | | | 2,0% |
| | | 0,04 | % Kaliumchlorid | | | | 0,2% |
| | | 77,46 | % Deionisiertes Wasser | | | | 2.7% |
| | | | | | | | |
| 10 | 2 | 3 | % Agar N700 | 57 | 38 | 23 | 12,5% |
| | | 15,75 | % Dextrin Crystal Tex 626 | | | | 67,9% |
| | | 0,75 | % Carrageen Gelcarin GP 812 | | | | 2,9% |
| | | 2,5 | % Glycerin | | | | 10,6% |
| | | 0,5 | % Gummi Arabicum | | | | 1,9% |
| | | 0,06 | % Kaliumchlorid | | | | 0,3% |
| | | 77,44 | % Deionisiertes Wasser | | | | 3,9% |
| | | | | | | | |
| 11 | 2 | 3 | % Agar N700 | 75 | 31 | 25 | 11,6% |
| | | 19,5 | % Dextrin Cleargum | | | | 75,68% |
| | | 2,5 | % Glycerin | | | | 9,7% |
| | | 0,003 | % Brilliantblau | | | | 0,01% |
| | | 0,002 | % Tartrazingelb | | | | 0,01% |
| | | 74,99 5 | % Wasser | | | | 3,0% |

| | | | | | | | |
|---|---|---|---|---|---|---|---|
| Agar N700 kann von C.E. Roeper GmbH unter dem Namen,CERO Agar Agar-quick soluble Agar N700 bezogen werden. | | | | | | | |
| Carraggeen Gelcarin kann von FMC Biopolymer bezogen werden. | | | | | | | |
| Dextrin Crystal Tex 626 kann von National Starch & Chemical bezogen werden. | | | | | | | |
| Dextrin Cleargum kann von Roquette GmbH bezogen werden. | | | | | | | |
| Modifizierte Stärke Perfectagel MPT kann von Avebe B.A. bezogen werden. | | | | | | | |

### Anmerkungen zu den einzelnen Beispielen:

Innerhalb der Gruppe der Beispiele 1 bis 3 sind die Beispiele 1 und 2 bevorzugt, da die erhaltenen Kapseln aufgrund des geringeren Glyceringehaltes in der Hüllmaterial-Zusammensetzung härter sind als im Beispiel 3. Dort bewirkt der höhere Glyceringehalt eine sehr weiche, feuchte Kapsel, die in der weiteren Verarbeitung von geringerer Stabilität ist als die Kapsel aus den Beispielen 1 und 2.

Die Kapseln aus den Beispielen 5 und 6 sowie aus den Beispielen 7 und 8 sind besonders stabile Kapseln; die jeweiligen Hüllmaterial-Zusammensetzungen sind besonders bevorzugt. Die Härtesteigerung gegenüber anders zusammengesetzten Kapseln geht auf die Anwesenheit der modifizierten Stärke Perfectagel MPT zurück (zur Bezugsquelle siehe oben). Die Kapselbruchstärken (in Newton) wurden zu den Beispielen 5 und 7 ermittelt. Der Wert für Beispiel 5 beträgt 3N, der für Beispiel 7 beträgt 4N.

Die Kapseln aus der Gruppe mit den Beispielen 9 und 10 sind von gleich guter Verarbeitbarkeit. Der höhere Carrageengehalt in Beispiel Nr. 10 führt jedoch zu einer Stabilitätssteigerung im direkten Vergleich mit der Kapsel aus Beispiel 9. Die Kapselbruchstärke für Beispiel 9 betrug 0,8N, die Kapselbruchstärke für Beispiel 10 betrug 1N.

Die Beispiele 1 bis 11 zeigen, dass sich unter Verwendung der erfindungsgemäßen Zusammensetzung stabile Kapseln mittels des Submerged Nozzle-Verfahrens herstellen lassen, wobei die Kapselhülle keine Gelatine umfasst. Die Anwesenheit der hydrolysierten Stärke mit Viskosität < 50 mPas ermöglicht eine hohe Trockensubstanz der Hülllösung; in den Beispielen wurden Trockensubstanzen im Bereich zwischen 23 und 25 Gewichtsprozent eingestellt, also Werte, die sich ganz allgemein als vorteilhaft erwiesen.

Die Viskosität der eingesetzten (erfindungsgemäßen) Hüllmaterial-Zusammensetzungen lag in den Beispielen im Bereich zwischen 26 und 100 mPas (gemessen bei 80 °C). Die Gelierpunkte der eingesetzten (erfindungsgemäßen) Hüllmaterial-Zusammensetzungen liegen in den Beispielen im Bereich zwischen 31 und 44 °C, also innerhalb eines besonders bevorzugten Bereiches.

## Patentansprüche

1. Zusammensetzung zur Verwendung als Hüllmaterial für die Herstellung einer nahtlosen Kapsel mit einem flüssigen Kern und einer diesen Kern umgebenden Hülle, umfassend:
- 1,5-4 Gew.-% Agar
- 10 - 22 Gew.-% einer hydrolysierten Stärke mit einer Viskosität < 50 mPas, gemessen bei 80 °C in wässriger Lösung mit einem Anteil von 15 Gew.-% an der hydrolysierten Stärke, bezogen auf die Gesamtmasse der wässrigen Lösung,
- 70 - 85 Gew.-% Wasser sowie
- gegebenenfalls einen oder mehrere übliche Zusatzstoffe,
und
wobei die Gewichtsprozentangaben; soweit nicht anders angegeben, auf die Gesamtmasse der Zusammensetzung bezogen sind.

2. Zusammensetzung nach Anspruch 1, wobei das Agar so zusammengesetzt ist, dass es eine Viskosität im Bereich zwischen 0,5 und 5 mPas besitzt, gemessen bei 80 °C in wässriger Lösung mit einem Anteil von 1 Gew.-% des Agar, bezogen auf die Gesamtmasse der wässrigen Lösung.

3. Zusammensetzung nach Anspruch 1 oder 2, wobei das Agar so zusammengesetzt ist, dass es eine Gelstärke im Bereich von 600 bis 900 g/cm² besitzt.

4. Zusammensetzung nach einem der vorangehenden Ansprüche, weiter umfassend
- 0,01 - 1 Gew.-% kappa-Carrageenan.

5. Zusammensetzung nach Anspruch 4, wobei das kappa-Carrageenan so zusammengesetzt ist, dass es eine Viskosität im Bereich zwischen 3 und 10 mPas besitzt, gemessen bei 80 °C in wässriger Lösung mit einem Anteil von 1 Gew.-% des kappa-Carrageenan, bezogen auf die Gesamtmasse der wässrigen Lösung.

6. Zusammensetzung nach einem der vorangehenden Ansprüche, **dadurch gekennzeichnet, dass** das Massenverhältnis Agar : sonstige Gelbildner im Bereich von 1,5 :1 bis 400 : 1 liegt.

7. Zusammensetzung nach einem der vorangehenden Ansprüche, **dadurch gekennzeichnet, dass** das Massenverhältnis Agar : kappa-Carrageenan im Bereich 3 : 1 bis 25 : 1 liegt.

8. Zusammensetzung nach einem der vorangehenden Ansprüche, weiter umfassend:
- 0,02 - 2 Gew.-% Gummi Arabicum.

9. Zusammensetzung nach einem der vorangehenden Ansprüche, wobei die Zusammensetzung eine Viskosität im Bereich zwischen 20 und 150 mPas besitzt, gemessen bei 80 °C.

10. Zusammensetzung nach einem der vorangehenden Ansprüche, weiter umfassend einen oder mehrere Süßstoffe und/oder Farbstoffe.

11. Zusammensetzung nach einem der vorangehenden Ansprüche, wobei die Zusammensetzung einen Gelierpunkt im Bereich zwischen 25 und 50°C besitzt.

12. Zusammensetzung nach einem der vorangehenden Ansprüche, weiter umfassend:
- 0,5 bis 10 Gew.-% Weichmacher.

13. Zusammensetzung nach einem der vorangehenden Ansprüche, weiter umfassend einen Weichmacher ausgewählt aus der Gruppe bestehend aus: Glycerin, Propylenglykol (1,2-Propandiol), Sorbitol, Mannitol und deren Mischungen.

14. Nahtlose Kapsel, umfassend einen flüssigen Kern und eine feste Kapselhülle, herstellbar in einem Mehrstoffdüsen-Verfahren mit in ein Härtungsbad eingetauchter Düse, in dem (a) eine flüssige Kernmischung und (b) als Hüllmaterial eine flüssige Zusammensetzung nach einem der vorangehenden Ansprüche eingesetzt werden.

15. Nahtlose Kapsel mit einem flüssigen Kern und einer diesen Kern umgebenden festen Hülle, wobei die Hülle umfasst:
- 5 bis 26 Gew.-% Agar
- 40 bis 75 Gew.-% einer hydrolysierten Stärke mit einer Viskosität < 50 mPas, gemessen bei 80 °C in wässriger Lösung mit einem Anteil, von 15 Gew.-% an der hydrolysierten Stärke, bezogen auf die Gesamtmasse der wässrigen Lösung,
- 0 bis 40 Gew.-% Weichmacher, sowie
- gegebenenfalls Restwasser und einen oder mehrere übliche Zusatzstoffe,
wobei die Gewichtsprozentangaben auf die Trockenmasse der Hülle bezogen sind.

16. Nahtlose Kapsel nach einem der Ansprüche 14 und 15, wobei der flüssige Kern (a) eine sensorisch wirksame Menge an einem oder mehreren Aromastoffen und/oder (b) eine pharmazeutisch wirksame Menge an einem oder mehreren pharmazeutischen Wirkstoffen umfasst.

17. Nahtlose Kapsel nach Anspruch 16, wobei der Anteil an Aromastoffen im flüssigen Kern zwischen 1 und 100 Gew.-% liegt, bezogen auf die Gesamtmasse des flüssigen Kerns.

18. Verwendung einer Zusammensetzung nach einem der Ansprüche 1 - 13 als Hüllmaterial in einem Mehrstoffdüsen-Verfahren mit eingetauchter Düse.

19. Verfahren zur Herstellung einer nahtlosen Kapsel nach einem der Ansprüche 14 - 17, mit folgenden Schritten:
- Bereitstellen eines flüssigen Kernmaterials,
- Bereitstellen einer flüssigen Zusammensetzung nach einem der Ansprüche 1 - 13,
- Simultanes Extrudieren des flüssigen Kernmaterials und der flüssigen Zusammensetzung durch die innere bzw äußere Düse einer Mehrstoffdüsenanordnung, so dass sich Tropfen bilden, die einen flüssigen Kern und eine flüssige Hülle besitzen,
- Härten der Tropfenhülle in einem Härtungsbad, so dass die Kapsel gebildet wird,
- gegebenenfalls Trocknen.

20. Verfahren nach Anspruch 19, wobei die Düse(n) der Mehrstoffdüsenanordnungen in das Härtungsbad eintauchen und dieses keine Kationen zur Aushärtung der flüssigen Zusammensetzung enthält.

## Claims

1. Composition for use as casing material for the preparation of a seamless capsule having a liquid core and a casing surrounding the core, comprising:
- from 1.5 to 4 wt.% agar,
- from 10 to 22 wt.% of a hydrolysed starch having a viscosity < 50 mPas, measured at 80°C in an aqueous solution having a content of 15 wt.% of the hydrolysed starch, based on the total mass of the aqueous solution,
- from 70 to 85 wt.% water and
- optionally one or more conventional additives,
and
wherein the percentages by weight, unless indicated otherwise, are based on the total mass of the composition.

2. Composition according to claim 1, wherein the composition of the agar is such that it has a viscosity in the range from 0.5 to 5 mPas, measured at 80°C in an aqueous solution having a content of 1 wt.% of the agar, based on the total mass of the aqueous solution.

3. Composition according to claim 1 or 2, wherein the composition of the agar is such that the agar has a gel strength in the range from 600 to 900 g/cm².

4. Composition according to any one of the preceding claims, further comprising
- from 0.01 to 1 wt.% kappa-carrageenan.

5. Composition according to claim 4, wherein the composition of the kappa-carrageenan is such that the kappa-carrageenan has a viscosity in the range from 3 to 10 mPas, measured at 80°C in an aqueous solution having a content of 1 wt.% of the kappa-carrageenan, based on the total mass of the aqueous solution.

6. Composition according to any one of the preceding claims, **characterised in that** the ratio by mass agar : other gel-forming agents is in the range from 1.5 : 1 to 400 : 1.

7. Composition according to any one of the preceding claims, **characterised in that** the ratio by mass agar : kappa-carrageenan is in the range from 3 : 1 to 25 : 1.

8. Composition according to any one of the preceding claims, further comprising:
- from 0.02 to 2 wt.% gum arabic.

9. Composition according to any one of the preceding claims, wherein the composition has a viscosity in the range from 20 to 150 mPas, measured at 80°C.

10. Composition according to any one of the preceding claims, further comprising one or more sweeteners and/or colourings.

11. Composition according to any one of the preceding claims, wherein the composition has a gel point in the range from 25 to 50°C.

12. Composition according to any one of the preceding claims, further comprising:
- from 0.5 to 10 wt.% plasticiser.

13. Composition according to any one of the preceding claims, further comprising a plasticiser selected from the group consisting of: glycerol, propylene glycol (1,2-propanediol), sorbitol, mannitol and mixtures thereof.

14. Seamless capsule comprising a liquid core and a solid capsule casing, which capsule can be prepared in a multiple nozzle process having a nozzle submerged in a hardening bath, in which process there are used (a) a liquid core mixture and (b) as casing material a liquid composition according to any one of the preceding claims.

15. Seamless capsule having a liquid core and a solid casing surrounding the core, wherein the casing comprises:
- from 5 to 26 wt.% agar,
- from 40 to 75 wt.% of a hydrolysed starch having a viscosity < 50 mPas, measured at 80°C in an aqueous solution having a content of 15 wt.% of the hydrolysed starch, based on the total mass of the aqueous solution,
- from 0 to 40 wt.% plasticiser and
- optionally residual water and one or more conventional additives,
wherein the percentages by weight are based on the dry mass of the casing.

16. Seamless capsule according to either claim 14 or claim 15, wherein the liquid core comprises (a) a sensorially effective amount of one or more flavourings and/or (b) a pharmaceutically effective amount of one or more pharmaceutical active ingredients.

17. Seamless capsule according to claim 16, wherein the amount of flavourings in the liquid core is from 1 to 100 wt.%, based on the total mass of the liquid core.

18. Use of a composition according to any one of claims 1 to 13 as casing material in a multiple nozzle process having a submerged nozzle.

19. Process for the preparation of a seamless capsule according to any one of claims 14 to 17, comprising the following steps:
- preparing a liquid core material,
- preparing a liquid composition according to any one of claims 1 to 13,
- simultaneously extruding the liquid core material and the liquid composition through the inner and outer nozzle, respectively, of a multiple nozzle arrangement so that drops having a liquid core and a liquid casing form,
- hardening the drop constituting the casing in a hardening bath so that the capsule is formed,
- optionally drying.

20. Process according to claim 19, wherein the nozzle(s) of the multiple nozzle arrangements are submerged in the hardening bath, and the hardening bath does not contain cations for hardening the liquid composition.

## Revendications

1. Composition destinée à être utilisée comme matériau d'enveloppe pour la fabrication d'une capsule sans couture avec un noyau liquide et une enveloppe entourant ce noyau, comprenant :
- 1,5 à 4 % en poids d'agar,
- 10 à 22 % en poids d'un amidon hydrolysé ayant une viscosité < 50 mPas, mesurée à 80°C en solution aqueuse avec une proportion de 15 % en poids d'amidon hydrolysé, par rapport à la masse totale de la solution aqueuse,
- 70 à 85 % en poids d'eau et
- le cas échéant un ou plusieurs additifs usuels,
et
- dans laquelle les indications de pourcentage en poids, sauf stipulation contraire, se rapportent à la masse totale de la composition.

2. Composition selon la revendication 1, dans laquelle l'agar est ainsi composé qu'il possède une viscosité dans la plage située entre 0,5 et 5 mPas, mesurée à 80°C en solution aqueuse avec une proportion de 1 % en poids d'agar, par rapport à la masse totale de la solution aqueuse.

3. Composition selon la revendication 1 ou 2, dans laquelle l'agar est ainsi composé qu'il possède une force en gel dans la plage de 600 à 900 g/cm².

4. Composition selon l'une des revendications précédentes, comprenant en outre
- 0,01 à 1 % en poids de kappa-carraghénane.

5. Composition selon la revendication 4, dans laquelle la kappa-carraghénane est ainsi composée qu'elle possède une viscosité dans la plage située entre 3 et 10 mPas, mesurée à 80°C en solution aqueuse avec une proportion de 1 % en poids de kappa-carraghénane, par rapport à la masse totale de la solution aqueuse.

6. Composition selon l'une des revendications précédentes, **caractérisée en ce que** le rapport en poids agar/ autres gélifiants se situe dans la plage de 1,5/1 à 400/1.

7. Composition selon l'une des revendications précédentes, **caractérisée en ce que** le rapport en poids agar/ kappa-carraghénane se situe dans la plage de 3/1 à 25/1.

8. Composition selon l'une des revendications précédentes, comprenant en outre
- 0,02 à 2 % en poids de gomme arabique.

9. Composition selon l'une des revendications précédentes, dans laquelle la composition possède une viscosité dans la plage située entre 20 et 150 mPas, mesurée à 80°C.

10. Composition selon l'une des revendications précédentes, comprenant en outre un ou plusieurs édulcorants et/ou colorants.

11. Composition selon l'une des revendications précédentes, dans laquelle la composition possède un point de gélification dans la plage située entre 25 et 50°C.

12. Composition selon l'une des revendications précédentes, comprenant en outre
- 0,5 à 10 % en poids de plastifiant.

13. Composition selon l'une des revendications précédentes, comprenant en outre un plastifiant choisi dans le groupe constitué de : glycérine, propylène glycol (1,2-propanediol), sorbitol, mannitol et leurs mélanges.

14. Capsule sans couture, comprenant un noyau liquide et une enveloppe de capsule solide, pouvant être fabriquée dans un procédé à buses multimatières avec une buse immergée dans un bain de durcissement, dans lequel on met en oeuvre (a) un mélange de noyau liquide et (b) une composition liquide à titre de matériau d'enveloppe selon l'une des revendications précédentes.

15. Capsule sans couture avec un noyau liquide et une enveloppe solide entourant ce noyau, dans laquelle la capsule comprend :
- 5 à 26 % en poids d'agar
- 40 à 75 % en poids d'un amidon hydrolysé ayant une viscosité < 50 mPas, mesurée à 80°C en solution aqueuse avec une proportion de 15 % en poids d'amidon hydrolysé, par rapport à la masse totale de la solution aqueuse,
- 0 à 40 % en poids de plastifiant, et
- le cas échéant, de l'eau résiduelle et un ou plusieurs additifs usuels,
dans laquelle les indications de pourcentage en poids se rapportent à la matière sèche de l'enveloppe.

16. Capsule sans couture selon une des revendications 14 et 15, dans laquelle le noyau liquide (a) comprend une quantité sensoriellement efficace d'un ou de plusieurs arômes et/ou (b) une quantité pharmaceutiquement efficace d'un ou de plusieurs principes actifs pharmaceutiques.

17. Capsule sans couture selon la revendication 16, dans laquelle la proportion d'arômes dans le noyau liquide se situe entre 1 et 100 % en poids, par rapport à la masse totale du noyau liquide.

18. Utilisation d'une composition selon l'une des revendications 1 à 13 à titre de matériau d'enveloppe dans un procédé à buses multimatières avec une buse immergée.

19. Procédé de fabrication d'une capsule sans couture selon l'une des revendications 14 à 17, comprenant les étapes suivantes :
- préparation d'une matière de noyau liquide,
- préparation d'une composition liquide selon l'une des revendications 1 à 13,
- extrusion simultanée de la matière de noyau liquide et de la composition liquide par la buse interne ou externe d'un dispositif à buses multimatières de sorte qu'il se forme des gouttelettes, qui possèdent un noyau liquide et une enveloppe liquide,
- durcissement de l'enveloppe de gouttelettes dans un bain de durcissement pour que des capsules se forment,
- le cas échéant séchage.

20. Procédé selon la revendication 19, dans lequel la ou les buses des dispositifs à buses multimatières plongent dans le bain de durcissement, celui-ci ne contenant aucun cation pour le durcissement de la composition liquide.
